# EUROPEAN PATENT APPLICATION

(11) **EP 2 228 091 A1**
(43) Date of publication of application: **15.09.2010**
(21) Application number: 09155029.3
(22) Date of filing: 12.03.2009
(51) Int. Cl.: A61M 25/00

(54) **Double lumen tubing with improved kinking resistance**

(71) Applicant: Joline GmbH & Co. KG, 72379 Hechingen (DE)
(72) Inventor: Steegers, Anselm, Dr., 72108 Rottenburg (DE); Schuler, Oliver, 72417 Jungingen (DE)
(74) Representative: Witte, Weller & Partner

(57) **Abstract**

Double lumen tubing (10) having a substantially circular cross-section and comprising a tubular wall (12), each extending about a longitudinal central axis of the tubing (12), and having a wall thickness being defined between the outer surface (14) and the inner surface (16). The tubing (10) comprises an inner wall (18) inside the tubing (10) for dividing it into a first (20) and a second (22) lumen, the inner wall (18), and each lumen (20;22) comprising a cross section with an arcuated portion (24;24') and a substantially plane portion (26;26') opposite to the arcuated portion (24;24'), the plane portions (26;26') of the lumens (20;22) lying adjacent to one another. Further, a centre region (30) of the arcuated portion (24;24') of the cross section of at least one of the first (20) or second (22) lumen, and the regions (39) where the tubular wall (12) meets the inner wall (18), have a wall thickness that is smaller than the wall thickness of two tubular wall regions (32;34) located left and right of said centre region (30).

## Description

The present invention relates to a double lumen tubing having a substantially circular cross section and comprising a tubular wall having an outer surface and an inner surface, each extending about a longitudinal central axis of the tubing. The tubing has a wall thickness which is defined between the outer surface and the inner surface, and the tubing comprises an inner wall inside the tubing for dividing it into a first and second lumen, each lumen being defined by the inner surface of the tubular wall and the inner wall, and each lumen comprising a cross section with an arcuated portion and a substantially plane portion opposite of the arcuated portion, the plane portions of the lumens lying adjacent to one another.

Double lumen tubings of this kind as generally known in the state of the art.

Such double lumen tubings are, e.g., used in medical devices which are used to provide access to vessels of a patient during, e.g. infusion and/or removal of body fluids. Catheters, which represent such tubings, have tapered distal tips to be percutaneously inserted into the patient. Double lumen catheters include a tubular wall that forms a fluid conduit which typically has a circular cross-section. Inside the tubing there is provided a divider wall which divides the lumen into two lumens. In many circumstances it is desirable to have two lumens with substantially equal cross-sectional areas; this is of particular importance where maximum fluid delivery rates through both lumens have to be guaranteed.

The catheter is inserted by means of guide wire that is first inserted to the later position of the catheter. Subsequently, the distal end of the catheter is moved over the guide wire and inserted into the position by means of the guide wire. As soon as the catheter is in its position, the guide wire is withdrawn. In the catheters, there may be provided a separate guide wire lumen for the guide wire in addition to the two lumens of the catheter, or one of the lumens is being temporarily used as the lumen for the guide wire and afterwards as lumen for withdrawing/delivering fluids to the body.

One of the most important features of such catheters is their kinking resistance, since upon kinking the catheter gets blocked and the withdrawal and/or the return of the fluid, e.g. blood, is obstructed. Thus, kinking may lead to complications and malfunctions during the operation of the medical devices which use a catheter of this kind.

In general, kinking might be avoided by means of increased wall thicknesses but, as an adverse effect, the lumen of a tubing designed in this way is impaired, i.e. a potential fluid flow through that tubing is adversely effected. Thus the ratio of the outer diameter i.e. the overall cross-section area of a tubing and the usable clearance, i.e. the lumen area is most important.

Many different approaches are known in the state of the art, which try to overcome the problem of the kinking resistance.

Many multilumen catheters of the prior art address the problem of kinking by including in the tube at least one lumen reinforced with a support member which may include a polymeric or metallic braid, coil or knit material, see, e.g., EP 1 144 039 A2.

Other approaches, like the one disclosed in US 2005/0277910 A1, try to overcome the problem of kinking by designing the catheter tube as a layered composite tube, whereby the interior that faces the lumen consists of a polyamide layer and the exterior consists of a polyurethane layer. These tubes are supposed to have improved characteristics in terms of resistance to kinking, a higher degree of rigidity during application and a reduced rigidity after application.

On the other hand, US 5,968,009 discloses a double lumen catheter having a supposedly increased kinking resistance. The lumen's central wall dividing the tubing's lumen into two lumens comprises an arcuate portion and has two surfaces, one bordering a first lumen, the other bordering the second lumen. Further, the first surface has a central convex arcuate portion, the second surface has a central concave arcuate portion, both portions having adjacent wing portions extending in the opposite directions to the tubular wall.

However, the catheters of the state of the art represent tubings which are not only complicated but also high in costs in view of their manufacturing.

Thus, and despite the different catheters addressing the improvement of the kinking resistance of its tubing, there still is a need for alternative tubings, with which an improved kinking resistance can be achieved whilst at the same time being easy to manufacture.

According to the invention, this problem is solved by modulating the wall thickness as a function of the circumferential angle. Thus the tubing at the outset is modulated such, that a region of the arcuated portion of the cross section of at least one of the first or second lumen, and the regions where the tubular wall meets the inner wall, has a wall thickness that is smaller than the wall thickness of two tubular wall regions located left and right of said centre region.

With the tubings's design according to the invention, the tubing overall gains a higher kinking resistance while at the same time achieve a favorable ratio of outer diameter to lumen area and, even more, being easy and cost-effective in its manufacturing. The tubing according to the invention has proven to have an improved kinking resistance values compared to other tubing solutions having different wall thicknesses. These improved characteristics are due to the fact that the arcuated portion of the tubular wall of the first and/or the second lumen, an its highest point of the arch, and lying perpendicular to the longitudinal axis of the tubing, and the regions where the tubular wall meets the inner wall, have a wall thickness that is smaller than the wall thickness of portions left and right to the highest point of the arch.

With the expression "located left and right" from the centre region, regions of the tubular wall are meant that are located on the tubular between the centre region and the regions where the tubular wall meets the inner wall.

Double lumen tubings as known in the art have - in general - a circular cross-section with a divider wall separating the tubing in two lumens each having a substantially semi-circular cross-section. With the tubing according to the invention, the cross-section of each of the lumens rather has - due to the different wall thicknesses in portions of the walls forming the lumens - the form of an isosceles triangle having chopped-off vertices, with the vertex formed by the meeting point of the two equal sides being more chopped-off than the other vertices.

In other words, the wall of each of the two lumens has, respectively at portions that are located from about 20° to about 80°, preferably about 45°, with respect to a virtual line perpendicularly cutting through the divider wall and the highest point of the arcuated portion, a wall thickness that is thicker compared to the wall portions forming the highest point of the of the arcuated portion and the divider wall.

In a preferred embodiment, the angle of the two equal angles of the isosceles triangle is about 45°.

According to another aspect of the invention, the centre region of the tubular wall as defined above has a wall thickness of about 40% to 80%, preferably of about 50%, of the tubular wall regions located left and right of said centre region.

With this measure, it is intended that, if the wall thickness of the tubular wall regions located left and right of the centre region is about 0,4 mm, the wall thickness of the centre region is about 0,2 mm, which would represent 50% of the wall thickness of the regions located left and right of the centre region.

According to other preferred embodiments of the invention, the two tubular wall regions located left and right of said centre region have a wall thickness of about 0,55 mm to about 0,80 mm.

According to a particular preferred embodiment, the centre region of the tubular wall has a wall thickness of about 0,50 mm, and the two tubular wall regions located left and right of said centre region have a wall thickness of about 0,64 mm.

In yet another preferred embodiment, the two tubular wall regions are each from about 10° to about 80° offset to the centre region, more preferably from about 30° to about 60° offset to said centre region, and most preferably about 45° offset to said centre region.

With these embodiments of the invention, double lumen tubings are provided that show a maximum kinking resistance, which is one of the most important features of tubings employed in medical applications.

In yet another embodiment, the cross-sectional areas of the first and the second lumens are substantially equal.

This measure has the advantage that the amount of blood flowing though the lumens and the blood flow characteristics in the lumens will be about the same.

The expression "substantially equal" as it is used herein, means that the cross-sections of the two lumens have the same shape and dimension with minor tolerances being possible which stem either from the manufacturing process or from an intended deviation. The cross-sectional areas are considered to be substantially equal in accordance with the invention if the larger of the two areas is no more than approximately 15% larger than the smaller one.

According to another embodiment of the invention, the cross-sectional areas of the first and the second lumen are different.

This measure has the advantage that a different flow amount and different flow characteristics in the lumens can be achieved which may be desirable in certain medical approaches.

According to a preferred embodiment, the tubing comprises flexible material, wherein it is preferred that the tubular wall and/or the inner wall comprises a flexible material.

A flexible material that may be used in this connection is any flexible homo- or copolymer, or polymer mixtures, which are commercially available and used in medical appliances. Thus, materials that can be used in the present invention comprise polyurethane, polyamide, silicone, polyethylene, polyamide or mixture thereof, or other materials with the same or similar characteristics as those just mentioned.

Of course, also non-flexible materials may be used with the present invention, e.g. metallic materials or alloys, such as stainless steel, cobalt-chromium alloys or others, in which case the catheter represents a rigid catheter, or comprises portions that are rigid in case flexible and non-flexible materials are used for the manufacture of the catheters.

Many different materials for use with a catheter are known in the state of the art, and it lies within the proficiency of a person skilled in the art to choose a material or a mixture of different materials for a certain application.

According to another embodiment of the invention, at least one of the first and the second lumen comprise a cross-sectional area of about 4 to about 6 mm², and more preferably if at least one of the first and the second lumen comprises a cross-sectional area of about 5,5 mm².

It is within the proficiency of a person skilled in the art to choose different or the same cross-sectional area of the lumens, depending of the respective application and vessel characteristics of the patient to be treated.

The double lumen tubing according to the invention may be preferably used as a catheter.

Thus, the invention also concerns the use of the catheter according to the invention in the withdrawing and delivery of fluids to a patient. The catheter according to the invention is particularly suited for use in hemodialysis.

It is to be understood, that the term "vessel" includes blood vessels, in particular veins.

Further advantages and features will become evident from the following description and from the attached drawing.

It will be appreciated that the aforementioned features and those still to be explained below can be used not only in the respectively cited combination but also in other combinations or on their own, without departing from the scope of the present invention.

### Brief description of the drawings

- Fig. 1: shows a cross-sectional view of a first preferred double lumen tubing in accordance with the present invention; and
- Fig. 2: shows views of longitudinal cuts (left hand, respectively) and a cross sections (right hand, respectively) through a portion of a double lumen catheter in accordance with the present invention (B) in comparison with double lumen tubings (A, C and D) not having the characteristics/dimensions of the tubing according to the invention.

The double lumen tubing according to the invention is designed for use as a catheter, and it may be used with any catheter device in any application where a double lumen catheter is needed.

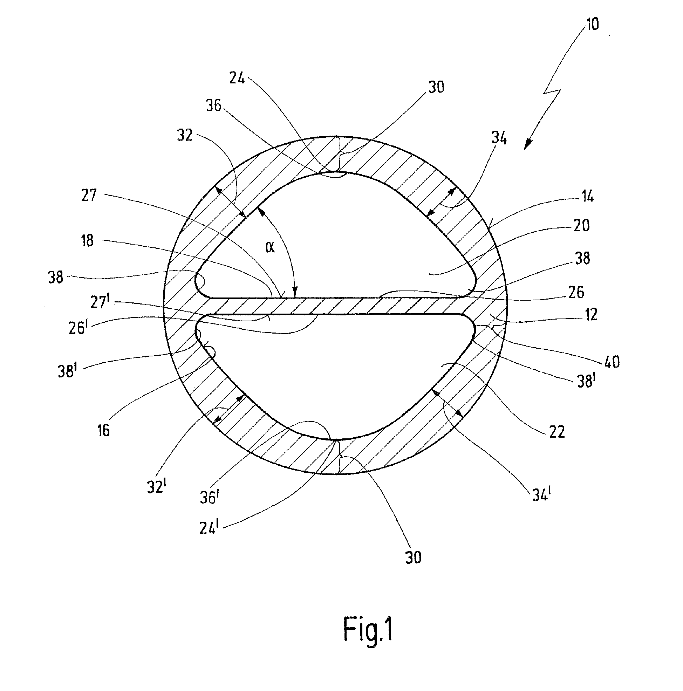

A first preferred embodiment according to the invention is shown in general with number 10 in Fig. 1. The double lumen tubing 10 includes a tubular wall 12, which has an outer surface 14 and an inner surface 16. The tubing 10 also comprises an inner or divider wall 18, dividing the lumen of the tubing 10 in two lumens, a first lumen 20 and a second lumen 22. Thus, each of the lumens 20 and 22 is defined by the inner surface 16 of the tubular wall 12 and the inner (or divider) wall 18.

The cross-sectional area of each of the lumens 20, 22 comprises an arcuated portion 24, 24' and a substantially plane portion 26, 26' opposite to the respective arcuated portion 24, 24', the plane portion 26, 26' being defined by the surface 27, 27' of inner wall 18, the arcuated portion 24, 24' being defined by the inner surface 16 of the tubular wall 12.

A centre region, indicated at arrow 30, 30' in Fig. 1, of the arcuated portion in the tubular wall 12 of the lumens 20, 22, perpendicular to the longitudinal axis of the tubing 10 and opposite to the plane portion 26, 26', has a wall thickness that is smaller than the wall thickness of two tubular wall regions located left and right of the centre region 30, 30', which are indicated with arrows 32 and 34 in Fig. 1. The thickness of the tubular wall 12 in the regions 39 where the inner wall 18 meets the tubular wall 12 is then, again, smaller than the thickness of the tubular wall regions 32 and 34, and can have about the thickness of the centre region 30, 30'.

In the embodiment as shown in Fig. 1, the centre region 30, 30' represents the highest point of the arcuated portion 24, 24' of the cross-sectional areas of the lumens 20, 22. Further, in the embodiment shown in Fig. 1, the wall regions 32; 34 left and right of the centre region 30, 30' of the arcuated portion, which have a larger thickness than the centre region 30, 30', are located about 45° with respect to a virtual line perpendicularly cutting through the inner wall 18 and the highest point of the arcuated portion, i.e. the centre region 30, 30'.

Further, and this can also be seen in the embodiment as shown in Fig. 1, the cross-sections of the lumens 20, 22 are about equal and have the shape of an isosceles triangle having chopped-off vertices 36, 36', 38, 38', with the vertex 36, 36' formed by the meeting point of the two equal sides being more chopped-off, and therefore being more rounded, than the other two vertices 38, 38'. In the embodiment shown in Fig. 1, the angle α of the two equal angles of the isosceles triangle is about 45°.

Thus, in a preferred embodiment, with the diameter of the tubing 12 being 5 mm - which represent 15 French - and with a wall thickness in the regions 32, 32', 34, 34' being about 0,64 mm and a wall thickness in the centre region 30, 30' of the tubular wall 12 being about 0,5 mm, a cross-sectional area of the lumens 20, 22 of about 5,5 mm² each may be reached.

Further, the wall thickness in the area where the inner wall 18 and the tubular wall meet each other, which is indicated by bracket 40 in Fig. 1, is about 0,45 mm. Also, in the embodiment shown in Fig. 1, the wall thickness of inner wall 18 is about 0,25 mm.

It lies within the proficiency of a person skilled in the art to transfer the dimensions exemplary given for a 15 French tubing to tubings having a smaller or a larger diameter.

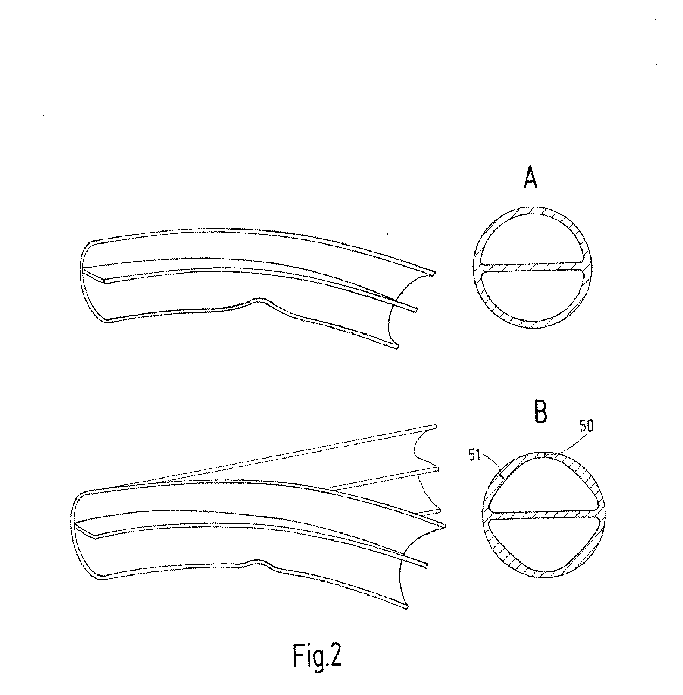

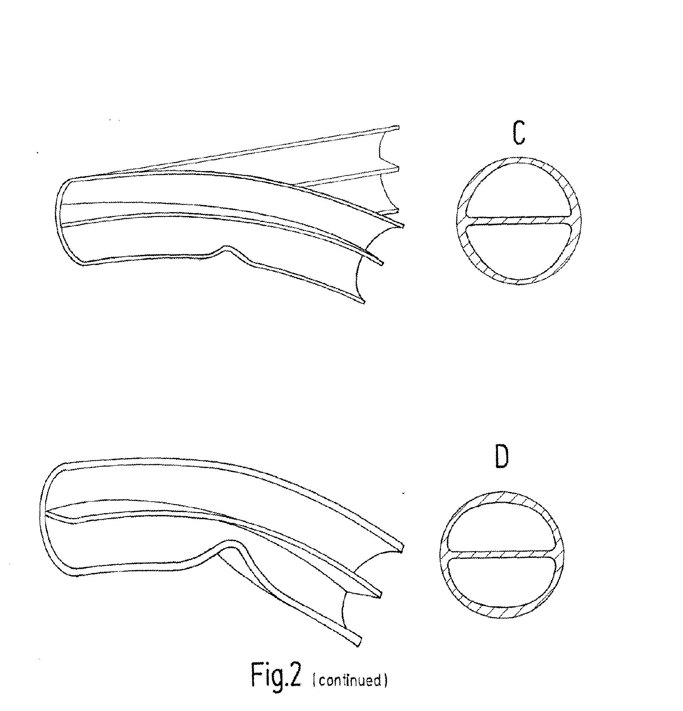

Fig. 2 represents schematic views of a tubing according to the invention (B) and other tubings (A, C, D) the kinking of which is being virtually simulated by means of a respective computer program.

The views presented in Fig. 2 show tubular cross-sections each of the same outer diameter and the same lumen areas but with different circumferential wall thickness distributions. As can be seen from Fig. 2, the tubing according to the invention (B) is much more resistant to kinking (see the simulated kinking view on the left hand side, respectively). The tubing according to the invention (B) has in the centre region of the tubular wall a smaller wall thickness than in regions left and right to that centre region, as indicated by the arrows 50, 51 (see cross-sectional view on the right hands, respectively). The tubular wall of the tubing as shown in A of Fig. 2 has an equal thickness; the tubular wall of the tubing as shown in C, although having a smaller thickness in the centre portion, it does not have also a smaller wall thickness in the region where the tubular wall meets the inner wall compared to the thickness of the tubular wall located left and right to the centre region. The tubular wall of the tubing as shown in D has a larger thickness in the centre region than in the regions left and right of the centre region.

## Claims

1. Double lumen tubing (10) having a substantially circular cross-section and comprising a tubular wall (12) having an outer surface (14) and an inner surface (16), each extending about a longitudinal central axis of the tubing (12), and having a wall thickness being defined between the outer surface (14) and the inner surface (16), the tubing (10) further comprising an inner wall (18) inside the tubing (10) for dividing it into a first (20) and a second (22) lumen, each lumen (20; 22) being defined by the inner surface (14) of the tubular wall (12) and the inner wall (18), and each lumen (20; 22) comprising a cross section with an arcuated portion (24; 24') and a substantially plane portion (26; 26') opposite to the arcuated portion (24; 24'), the plane portions (26; 26') of the lumens (20; 22) lying adjacent to one another, **characterized in that a** centre region (30) of the arcuated portion (24; 24') of the cross section of at least one of the first (20) or second (22) lumen, and the regions (39) where the tubular wall (12) meets the inner wall (18), have a wall thickness that is smaller than the wall thickness of two tubular wall regions (32; 34) located left and right of said centre region (30).

2. Double lumen tubing (10) according to claim 1, **characterized in that** said centre region (30) of the tubular wall (12) has a wall thickness of about 40% to 80%, preferably of about 50 %, of the tubular wall regions (32; 34) located left and right of said centre region (30).

3. Double lumen tubing (10) according to claim 1 or 2, **characterized in that** said two tubular wall regions (32; 34) located left and right of said centre region (30) have a wall thickness of about 0,55 mm to about 0,80 mm.

4. Double lumen tubing (10) according to any of claims 1 to 3, **characterized in that** said centre region (30) of the tubular wall (12) has a wall thickness of about 0,50 mm and said two tubular wall regions (32; 34) located left and right of said centre region (30) have a wall thickness of about 0,64 mm.

5. Double lumen tubing (10) according to any of the foregoing claims, **characterized in that** said two tubular wall regions (32; 34) are each from about 10° to about 80° offset to the centre region (30).

6. Double lumen tubing (10) according to claim 5, **characterized in that** said two tubular wall regions (32; 34) are from about 30° to about 60° offset to said centre region (30).

7. Double lumen tubing (10) according to claim 6, **characterized in that** said two tubular wall regions (32; 34) are about 45° offset to said centre region (30).

8. Double lumen tubing (10) according to any of the preceding claims, **characterized in that** the cross-sectional areas of the first (20) and the second (22) lumen are substantially equal.

9. Double lumen (10) tubing according to one of claims 1 to 7, **characterized in that** the cross-sectional areas of the first (20) and the second (22) lumen are different.

10. Double lumen tubing (10) according to any of the preceding claims, **characterized in that** the tubing (10) comprises a flexible material.

11. Double lumen tubing (10) according to any of the preceding claims, **characterized in that** the tubular wall (12) and/or the inner wall (18) comprises a flexible material.

12. Double lumen tubing (10) according to any of the preceding claims, **characterized in that** at least one of the first (20) and the second (22) lumen comprise a cross-sectional area of about 4 to about 6 mm².

13. Double lumen tubing (10) according to claim 12, **characterized in that** at least one of the first (20) and the second (22) lumen comprise a cross-sectional area of about 5,5 mm².

14. Double lumen tubing (10) according to any of the preceding claims for use as a catheter.
